(19) Europäisches Patentamt
European Patent Office
Office européen des brevets

(11) **EP 4 299 063 B1**

(12) **EUROPEAN PATENT SPECIFICATION**

(45) Date of publication and mention
of the grant of the patent:
**01.05.2024 Bulletin 2024/18**

(21) Application number: **22382623.1**

(22) Date of filing: **30.06.2022**

(51) International Patent Classification (IPC):
***A61K 31/40*** (2006.01)   ***A61K 9/20*** (2006.01)
***A61K 9/48*** (2006.01)

(52) Cooperative Patent Classification (CPC):
**A61K 9/4808; A61K 9/2009; A61K 31/40**

(54) **ORAL CAPSULES COMPRISING ATORVASTATIN TABLETS SHOWING SUITABLE DISSOLUTION PROFILE AND BIOAVAILABILITY**

ORALE KAPSELN, DIE ATORVASTATIN-TABLETTEN UMFASSEN UND EIN GEEIGNETES AUFLÖSUNGSPROFIL UND EINE GEEIGNETE BIOVERFÜGBARKEIT AUFWEISEN

CAPSULES À AVALER COMPRENANT DES COMPRIMÉS D'ATORVASTATINE PRÉSENTANT UN PROFIL DE DISSOLUTION ET UNE BIODISPONIBILITÉ ADAPTÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.01.2024 Bulletin 2024/01**

(73) Proprietor: **Ferrer Internacional, S.A.**
**08029 Barcelona (ES)**

(72) Inventors:
• **FERNÁNDEZ MOLLAR, Berta**
**08173 SANT CUGAT DEL VALLÈS (ES)**

• **URBANO HURTADO, Javier**
**08173 SANT CUGAT DEL VALLÈS (ES)**

(74) Representative: **ZBM Patents - Zea, Barlocci & Markvardsen**
**Rambla Catalunya, 123**
**08008 Barcelona (ES)**

(56) References cited:
**EP-A1- 3 184 103      WO-A1-2014/195421**
**WO-A1-2021/145676**

**Description**

**Technical Field**

The present invention relates to oral pharmaceutical compositions, specifically to oral capsules comprising at least one atorvastatin tablet and to atorvastatin tablets suitable to be placed inside capsules.

**Background Art**

Capsules are a well-known pharmaceutical dosage form. They are usually taken orally but can also be used as a reservoir for alternative administration routes like the inhalation or rectal routes. Capsules may be hard-shelled capsules, usually containing solids, or soft-shelled capsules, primarily used for oils and for active ingredients that are dissolved or suspended in oil.

Hard-shelled capsules may contain tablets (including minitablets), dry powdered ingredients, granulates, pellets or beads or combinations thereof. Hard-shelled capsules are made in two halves: a smaller-diameter "body" that is filled and then closed using a larger-diameter "cap".

Capsules are made from aqueous solutions of gelling agents, such as animal protein (mainly gelatin) or plant polysaccharides or their derivatives (such as carrageenans and modified forms of starch and cellulose, such as hydroxypropyl methylcellulose -HPMC-). Other ingredients can be added to the gelling agent solution including plasticizers such as polyethyleneglycol (PEG), glycerol, sorbitol, propyleneglycol, PEO-PPO block copolymers and other polyalcohols and polyethers to decrease the capsule's hardness, coloring agents, preservatives, disintegrating agents, lubricants and surface treatments.

Hard-shelled capsules are useful for administering several active ingredients at the same time. These active ingredients may be incorporated for instance in the form of tablets (including minitablets), dry powdered ingredients, granulates, pellets, beads, or combinations thereof.

When active agents are incorporated as tablets, tablets are usually piled up as shown for instance in Figure 1 of WO 2009/118359 A2, included here as Figure 10.

Hard-shelled capsules useful for oral administration can have a wide range of sizes. Although there are larger capsules disclosed, typical sizes range from size '000' to size '5' capsules.

The volume, the locked length (the outer length between the ends of the capsule when body and cap are placed together measured from the top of the curved part) and the external diameter of the body (the body is the part having the smaller diameter compared to the cap) are tabulated below for some capsules sizes marketed by a major capsule provider for illustrative purposes only.

| Size | Volume (mL) | Locked length (mm) | Ext. body diameter (mm) |
|------|-------------|--------------------|-------------------------|
| 5 | 0.13 | 11.1 | 4.68 |
| 4 | 0.21 | 14.3 | 5.05 |
| 3 | 0.30 | 15.9 | 5.57 |
| 2 | 0.37 | 18.0 | 6.07 |
| 1 | 0.50 | 19.4 | 6.63 |
| 0 | 0.68 | 21.7 | 7.34 |
| 0E | 0.78 | 23.5 | 7.34 |
| 00 | 0.91 | 23.3 | 8.18 |
| 000 | 1.37 | 26.1 | 9.55 |

As an example, size '0' capsules have an intermediate size which is convenient for patients because capsules are not too large to be swallowed and enable to place several tablets of reduced size inside.

The size of the tablets to be placed inside a size '0' capsules need to have a certain tolerance in relation to the internal diameter of the body for manufacturing purposes (for instance, to avoid tablets being stuck in the inner walls of the capsule preventing the incorporation of other tablets). Usually, tablets having a maximum diameter of around 6 mm (or 6.5 mm) can be placed inside. The place available is limited by the internal diameter of the body of the capsule, which, for obvious reasons, is below the value of the external diameter (here 7.34 mm) due to the thickness of the capsule itself.

Regarding the number of tablets that can be placed inside a capsule, one should appreciate that the locked length is the outer length of the ends of the capsule when closed measured from the top of the curved part. Therefore, there is significantly less height inside than the value of the locked length to place tablets piled up. Additionally, the shape of the tablet, for example a flat or biconvex round tablet, may not fit with the curved part of the capsule, thus reducing the effective height available for the tablets. Considering again size '0' capsules, the sum of the thickness of tablets should ideally be below 17 mm to accommodate in a locked length of 21.7 mm. This means that if for instance 5 tablets are to be placed inside a size '0' capsule, the sum of their thicknesses should ideally be below 17 mm. In addition, when filling capsules the body of the capsule (which is shorter than the locked length) is filled and then closed with the cap. Thus, the total height of the tablets piled up, should ideally not be higher than the length of the body to avoid those tablets leave the capsule in the process.

All in all, for capsules comprising several tablets inside, in order to have a convenient size for patients, tablets placed inside need to have a reduced diameter and thickness. This need limits the amount of active ingredient to be placed in such tablets and reduces the amounts of excipients that can be placed, which, in turn, may reduce the dissolution rate and the bioavailability of the active agent of such tablets placed inside a capsule. These problems are more significant for active ingredients showing poor solubility.

Atorvastatin and its salts show poor aqueous solubility and high permeability and are thus classified as class II in the Biopharmaceutics Classification System (BCS). Poor aqueous solubility affects the bioavailability of orally administered drugs.

The solubility of the drug is one of its most important physicochemical properties in drug release and absorption, hence, playing an integral role in the bioavailability of an orally administered drug.

Tablets comprising atorvastatin or its salts are commercially available. In these commercial tablets, atorvastatin or its salts, especially calcium atorvastatin, is generally found comprising about 10 % weight/weight of total weight of the tablet. For instance, Cardyl® 80 mg film-coated tablets contain 86.6 mg of calcium atorvastatin trihydrate and have a total weight of 830 mg. Thus, the atorvastatin salt is present in 10.4 % weight/weight (% w/w) in relation to the total weight of the atorvastatin tablet. Excipients are known to improve the disintegration and dissolution speed of atorvastatin and its salts and for this reason commercial tablets tend to contain atorvastatin in low % weight/weight to favor its disintegration and dissolution. Atorvastatin generic tablets produced by other companies also have a low % weight/weight to favor its dissolution. For instance, Atorvastatina Cinfa 80 mg comprimidos recubiertos (atorvastatin 80 mg coated tablets) or Atorvastatina TEVAGEN 80 mg (atorvastatin 80 mg coated tablets) contain respectively 7.7 and 10.5 % w/w of atorvastatin calcium in relation to the total weight of the atorvastatin tablet.

In WO 2014/195421 A1 capsules including separate tablets comprising: a) an HMG-CoA reductase inhibitor (a 'statin'), in particular, atorvastatin or rosuvastatin or salts thereof, and b) acetylsalicylic acid (aspirin) in a manner to minimize interaction of acetylsalicylic acid with the statin were disclosed. According to this document, the statin tablets show good stability while maintaining a relative fast release of aspirin. This effect was achieved by a specific type of coating applied to aspirin tablets at a specific range of coating thickness and by using at least two aspirin tablets to achieve a fast release profile. In the examples, capsules also include tablets comprising ramipril.

The capsules disclosed in Examples 1 and 2 of WO 2014/195421 A1 contain two atorvastatin tablets of 10 or 20 mg (see Table 1 on page 19 of WO 2014/195421 A1).

The 10 mg atorvastatin tablet comprises 10.845 mg of atorvastatin calcium trihydrate in a total weight of 113 mg per coated tablet, i.e., 9.4 % w/w of atorvastatin (see Table 2 on pages 19-20) in relation to the total weight of the coated tablet.

The 20 mg atorvastatin tablet comprises 21.69 mg of atorvastatin calcium trihydrate in a total weight of 144.20 mg per coated tablet, i.e., 15.0 % w/w of atorvastatin (see Table 3 on page 20) in relation to the total weight of the coated tablet.

The amount of Polysorbate 80, a non-ionic surfactant, is below 1.4 % w/w in relation to the total weight of the coated tablet both for the 10 and 20 mg atorvastatin tablets.

The dissolution profile was only disclosed for the aspirin coated tablets. In this regard, in order to accelerate the dissolution profile of aspirin, two 50 mg tablets having a higher percentage of excipients instead of one single 100 mg tablet were used. However, In WO 2014/195421 A1 no information on the dissolution profile or bioavailability of atorvastatin tablets was available.

If one active ingredient shows poor dissolution, as is the case here with atorvastatin and pharmaceutically acceptable salts thereof, based on the dissolution profile of capsules comprising atorvastatin tablets alone, it is extremely difficult to predict oral bioavailability of them when such products are compared to a reference product at the standard buffers and Quality Control media (for the USA, pH 1.0, 4.5 and 6.8). Placing atorvastatin tablets in capsules further complicates the development, as the release of active ingredients (among them atorvastatin) is further delayed in time by the dissolution of the capsule. Additionally, the size of capsules is limited, thus limiting the size of the tablets that can be placed inside the capsule. All in all, the development of tablets to be placed inside a capsule is much more complex than the development of conventional tablets due to the implicit limitations related to the capsule. At the same time atorvastatin tablets should be stable.

Thus, from what is known in the art, there is still the need of preparing atorvastatin tablets that can be placed in a capsule of reasonable size for patients, wherein in such capsule atorvastatin or pharmaceutically acceptable salts thereof can show bioavailability comparable to that of approved reference products.

**Summary of the Invention**

The present inventors have developed atorvastatin tablets which comprise granulated calcium carbonate and non-ionic surfactant in specific amounts wherein atorvastatin is present in high percentages. These tablets can be placed in capsules of reasonable size for patients, which show bioavailability comparable to that of approved reference products, such as Cardyl® film-coated tablets. Showing comparable bioavailability to approved reference products is critical because this enables the regulatory approval by medicine agencies of such products by showing bioequivalence regarding such reference products. In this way further unneeded, lengthy, and costly safety and efficacy studies can be avoided. Thus, patients are not exposed to unnecessary clinical studies in order to approve medicines according to the invention.

Moreover, this effect is unexpected because of the poor dissolution of atorvastatin and pharmaceutically acceptable salts thereof, based on the dissolution profile of capsules comprising atorvastatin tablets alone. Regarding this, the similarity factor (f2) disclosed in the examples for the capsules of the invention showed a dissolution profile not comparable to that of the reference product. Dissolution profiles were significantly delayed compared to the reference product at all pH values tested. However, surprisingly, the capsules of the invention showed similar bioavailability to that of the reference product.

In addition, the dissolution profile was surprisingly accelerated at pH 6.8 for the capsules of the invention comprising granulated calcium carbonate with respect to the use of standard calcium carbonate (see examples section).

On the other hand, the atorvastatin tablets of the present invention also enable to place higher doses of atorvastatin in fewer tablets than those that would be required by using the tablets of the prior art or just one tablet and at the same time show suitable bioavailability. This enables to place for instance a dose of 20 or 40 mg in just one small tablet or the dose of 80 mg of atorvastatin in no more than two tablets in a capsule of size '0' or to provide for more space for further ingredients of the capsule. This also enables to place atorvastatin tablets together with tablets of other active ingredients, such as in cardiovascular polypills as those disclosed in WO 2014/195421 A1, in capsules of a size acceptable for patients.

Accordingly, the present invention relates to an oral capsule comprising at least one atorvastatin tablet, wherein such atorvastatin tablets comprise:

a) an amount of 25-35 % weight/weight of atorvastatin or pharmaceutically acceptable salts thereof,

b) an amount of 2-3 % weight/weight of a non-ionic surfactant, and

c) an amount of 20-35 % weight/weight of granulated calcium carbonate,

wherein all amounts in % weight/weight are calculated in relation to the total weight of the atorvastatin tablet.

**Brief description of the drawings**

Figure 1 shows the dissolution profiles of tablets of Cardyl® 80 mg film-coated tablets, Examples 1-3 and Comparative Examples 1-3 tablets at pH=1 at 50 rpm and 900 mL.

Figure 2 shows the dissolution profiles of Cardyl® 80 mg film-coated tablets, Examples 1-3 and Comparative Examples 1-3 tablets at pH 4.5 at 50 rpm and 900 mL

Figure 3 shows the dissolution profiles of Cardyl® 80 mg film-coated tablets, Examples 1-3 and Comparative Examples 1-3 tablets at pH 6.8 at 50 rpm and 900 mL.

Figure 4 shows the dissolution profiles of Cardyl® 80 mg film-coated tablets, Example 4 and Comparative Example 4 tablets at pH 6.8 at 50 rpm and 900 mL.

Figure 5 shows the dissolution profiles of Example 4 Capsules and Comparative Example 4 Capsules at pH=6.8 at 75 rpm and 900 mL.

Figure 6 shows the dissolution profiles of Example 1 Capsules and Comparative Example 1 Capsules at pH= 6.8 at 75 rpm and 900 mL.

Figure 7 shows the dissolution profiles of Example 1 Capsules, Example 4 Capsules, Comparative Example 3 Capsules, and Comparative Example 4 Capsules at pH= 1.0, at 50 rpm and 900 mL.

Figure 8 the dissolution profiles of Example 1 Capsules, Example 4 Capsules, Comparative Example 3 Capsules, and Comparative Example 4 Capsules at pH= 4.5 at 50 rpm and 900 mL.

Figure 9 the dissolution profiles of Example 1 Capsules, Example 4 Capsules, Comparative Example 3 Capsules, and Comparative Example 4 Capsules at pH= 6.8, at 75 rpm and 900 mL.

Figure 10 shows a capsule comprising tablets.

## Detailed description of the invention

As mentioned above, the present invention relates to an oral capsule comprising at least one atorvastatin tablet, wherein such atorvastatin tablets comprise: a) an amount of 25-35 % weight/weight of atorvastatin or pharmaceutically acceptable salts thereof, b) an amount of 2-3 % weight/weight of a non-ionic surfactant, and c) an amount of 20-35 % weight/weight of granulated calcium carbonate, wherein all amounts in % weight/weight are calculated in relation to the total weight of the atorvastatin tablet.

As used herein the term "tablet" includes tablets, mini tablets or micro tablets. Similarly, the term "capsule" also can refer to micro capsules. Suitable capsules may be either hard- or soft-shelled capsules. Capsules may be made of gelatin, starch, or a cellulosic material, such as HPMC capsules, hard-shelled gelatin capsules being preferred. Two-piece hard-shelled gelatin capsules could optionally be sealed by gelatin bands or the like or just closed.

For the purposes of the invention, any ranges given include both the lower and the upper endpoints of the range.

As used herein, the terms "aspirin" or "acetylsalicylic acid" are used interchangeably.

The expression "pharmaceutically acceptable salts" includes both "pharmaceutically acceptable cationic salts" and "pharmaceutically acceptable acid addition salts".

The expression "pharmaceutically acceptable cationic salts" is intended to define but is not limited to such salts as the alkali metal salts (e.g. sodium and potassium), alkaline earth metal salts (e.g. calcium and magnesium), aluminum salts, ammonium salts, or salts with organic amines such as benzathine (N,N'- dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol), or procaine.

The expression "pharmaceutically acceptable acid addition salts" is intended to define but is not limited to such salts as the hydrochloride, hydrobromide, sulfate, hydrogen sulfate, phosphate, hydrogen phosphate, dihydrogen phosphate, acetate, succinate, citrate, methanesulfonate (mesylate), or p-toluenesulfonate (tosylate) salts.

The pharmaceutically acceptable cationic salts of atorvastatin containing free carboxylic acid may be readily prepared by reacting the free acid form of atorvastatin with an appropriate base, usually one equivalent, in a solvent or solvent system. Typical bases are sodium hydroxide, potassium hydroxide, sodium methoxide, sodium ethoxide, sodium hydride, potassium methoxide, magnesium hydroxide, calcium hydroxide, benzathine, choline, diethanolamine, piperazine and tromethamine. The salt can be isolated by concentration to dryness or by addition of a non-solvent. In many cases, salts are preferably prepared by mixing a solution of the acid with a solution of a different salt of the cation (sodium or potassium ethylhexanoate, magnesium oleate), employing a solvent (e.g., ethyl acetate) from which the desired cationic salt precipitates, or can be otherwise isolated by concentration and/or addition of a non-solvent. The pharmaceutically acceptable acid addition salts of atorvastatin containing free amine groups may be readily prepared by reacting the free base form of atorvastatin with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However, when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates or can be otherwise isolated by concentration and/or addition of a non-solvent.

Atorvastatin or its salts may be in crystalline form either as free solvation compounds or as solvates (e.g., hydrates) and it is intended that both forms are within the scope of the present invention. Methods of solvation are generally known within the art.

The term "solvate" refers to a molecular complex comprising the atorvastatin or a salt thereof, and a stoichiometric or non-stoichiometric amount of one or more solvent molecules bound by non-covalent intermolecular forces.

When the one or more solvent molecules forming part of the molecular complex is water, the solvate is a hydrate.

Atorvastatin can also be in amorphous form for the purposes of the present invention. In a particular embodiment, in combination with any of the embodiments of the invention, the oral capsule of the invention is that in which atorvastatin or pharmaceutically acceptable salts thereof are in crystalline form.

In another particular embodiment, in combination with any of the embodiments of the invention, the oral capsule of the invention is that in which atorvastatin is in the form of a calcium or magnesium salt of atorvastatin.

In another particular embodiment, in combination with any of the embodiments of the invention, the oral capsule of the invention is that in which the atorvastatin tablet is prepared using crystalline calcium atorvastatin trihydrate.

As used herein the term 'Dv90' is the maximum particle diameter below which 90% of the sample volume exists. According to the present invention, Dv90 is measured by laser diffraction. The Dv90 equivalent spherical volume diameter thus found is a statistical representation of the 90% point on a cumulative frequency plot. The Dv90 equivalent sphere volume diameters of the particles of the compound (atorvastatin or its salts) is evaluated using a Malver Mastersizer 2000 Particle Size Distribution Analyzer or other such equipment recognized by those skilled in the art. Using such instrument values for a suspension of the particles of unknown size are obtained, and the instrument is monitored using a control sample having particles within the size range expected based on statistical analysis of the control sample. The particle size of the compound prior to formulation into a pharmaceutical composition can be measured, for example, as follows. The laser scattering particle size distribution analysis is conducted on a small sample of the reduced material, which is suspended in approximately 180 ml of dispersant solution. Prior to sample suspension, a dispersant solution containing 0.1% SPAN 80 in cyclohexane, and pre saturated with the compound is prepared. The dispersant solution is filtered through a 0.2 micron microporous membrane filter to provide a particle-free dispersant solution. The sample then is added to the dispersant solution until an acceptable level of laser light obscuration is achieved, at which point the particle size distribution is measured. Triplicate measurements are conducted as a minimum a) to provide more reliable measurements and b) to check the equivalent sampling of the suspended material. The results are automatically recorded and displayed graphically to give a cumulative % undersize vs. diameter and a frequency percentage vs. diameter for the sample. From this, the d90 equivalent spherical volume diameter value (Dv90) is derived (90% cumulative undersize value).

In a particular embodiment, in combination with any of the embodiments of the invention, the oral capsule of the invention is that in which atorvastatin or pharmaceutically acceptable salts thereof shows Dv90 < 50 microns.

The term 'granulated calcium carbonate' is understood to be calcium carbonate granulated with a binder such as maize starch, potato starch, pregelatinized starch, maltodextrin, sorbitol, polyvinyl pyrrolidone PVP), acacia gum, methylcellulose, hydroxypropyl cellulose (HPC), hydroxypropyl methylcellulose (HPMC), sodium alginate (Na-Alg), gelatin, syrup, polyethylene glycol (PEG), Eudragit® E 100 (a cationic copolymer based on dimethylaminoethyl methacrylate, butyl methacrylate, and methyl methacrylate with a ratio of 2:1:1), or mixtures thereof. Calcium carbonate is preferably granulated with binders through wet granulation.

Granulated calcium carbonate may be characterized by a particle size distribution in which 8 % or less of the weight of the granules show a diameter of 500 microns or larger and 85 % or more of the weight of the granules show a diameter larger than or equal to 80 microns, both measured by analytical sieving. Preferably, 5 % or less of the weight of the granules of granulated calcium carbonate show a diameter of 500 microns or larger and 90 % or more of the weight of the granules show a diameter larger than or equal to 80 microns, both measured by analytical sieving.

Particle size distribution is estimated by analytical sieving by mechanical agitation (dry sieving method). Procedure: Tare each test sieve to the nearest 0.1 g. Place an accurately weighed quantity of test sample on the top (coarsest) sieve and replace the lid. Agitate the nest of sieves for 5 min using vibrations with an amplitude of vibration of 2 mm and a frequency of 50 Hz, then carefully remove each sieve from the nest without loss of material. Reweigh each sieve and determine the mass of material on each one. Determine the mass of material in the collecting pan in a similar manner.

Bulk densities of granulated calcium carbonate useful for the invention usually range from 0.5 to 0.9 g/mL, preferably from 0.55 to 0.85 g/mL. Bulk densities are measured according to method 1 of the European Pharmacopoeia, 10.7, section '2.9.34. Bulk density and tapped density of powders' by carrying out 500 and 1250 taps on the same powder sample and reading the corresponding volumes V500 and V1250 to the nearest graduated unit.

Granulated calcium carbonate is available as a commercial excipient for instance with the range of products marketed under the tradename Scoralite® DC, by SCORA S.A.S., including calcium carbonate granulated with different binders. Scoralite® DC range of products are designed to be used for direct compression and includes: Scoralite® DC 90ST & 95ST, with 10% and 5% maize starch, Scoralite® DC 90pST, with 10% pregelatinized starch, Scoralite® DC 90MD & 95MD, with 10% and 5% maltodextrin, Scoralite® DC SO, with sorbitol, Scoralite® DC 97PVP, with 3% polyvinylpyrrolydone, Scoralite® DC90 & 95AC, with 10% and 5% acacia gum.

In a particular embodiment, in combination with any of the embodiments of the invention, the oral capsule of the invention comprises at least one atorvastatin tablet comprising granulated calcium carbonate, in which the granulated calcium carbonate is calcium carbonate granulated with a binder in an amount equal to or less than 20 % weight/weight, wherein the % weight/weight is calculated in relation to the total weight of granulated calcium carbonate.

In another particular embodiment, in combination with any of the embodiments of the invention, the oral capsule of the invention comprises at least one atorvastatin tablet comprising granulated calcium carbonate, in which the granulated calcium carbonate comprises an amount of binder equal to or less than 10 % weight/weight, wherein the % weight/weight is calculated in relation to the total weight of granulated calcium carbonate.

In another particular embodiment, in combination with any of the embodiments of the invention, the oral capsule of the invention comprises at least one atorvastatin tablet comprising granulated calcium carbonate, in which the granulated calcium carbonate is calcium carbonate granulated with a binder selected from: maize starch, pregelatinized starch, potato starch, maltodextrin, sorbitol, polyvinyl pyrrolidone (PVP), acacia gum, methylcellulose, hydroxypropyl cellulose (HPC), hydroxypropyl methylcellulose (HPMC), sodium alginate (Na-Alg), gelatin, syrup, polyethylene glycol (PEG), a cationic copolymer based on dimethylaminoethyl methacrylate, butyl methacrylate and methyl methacrylate with a ratio of 2:1:1, and mixtures thereof.

In another particular embodiment, in combination with any of the embodiments of the invention, the oral capsule of the invention comprises at least one atorvastatin tablet comprising granulated calcium carbonate, in which the granulated calcium carbonate is calcium carbonate granulated with starch, pregelatinized starch or mixtures thereof. In a further embodiment, the granulated calcium carbonate comprises an amount of starch, pregelatinized starch or mixtures thereof equal to or less than 20 % weight/weight, wherein the % weight/weight is calculated in relation to the total weight of the granulated calcium carbonate. In a further embodiment, in combination with any of the embodiments of the invention, the oral capsule of the invention comprises at least one atorvastatin tablet comprising granulated calcium carbonate which comprises an amount of starch, pregelatinized starch or mixtures thereof equal to or less than 10 % weight/weight. In a further embodiment, the granulated calcium carbonate comprises an amount of starch or pregelatinized starch of 5-10 % weight/weight. In the embodiments of this paragraph the % weight/weight is calculated in relation to the total weight of the granulated calcium carbonate.

A non-ionic surfactant is an amphiphilic molecule having a hydrophobic part and a hydrophilic part, which allows it to be a surface-active molecule, that is substantially nonionized (*i.e.* uncharged) in water in neutral pH.

Suitable non-ionic surfactants for the present invention include, but are not limited to, block copolymers of ethylene and propylene oxide, fatty acid esters, medium-chain triglycerides, cyclodextrins, polyethylene glycols, sorbitan alkyl esters, polyoxyethylene sorbitan fatty acid esters (i.e. polysorbates), polyoxylglycerides, polyoxyethylene fatty alcohol ethers, polyoxyethylene castor oil derivatives, polyoxyethylene alkyl esters, polyvinyl caprolactam-polyvinyl acetate-polyethylene glycol graft copolymer, polypropylene glycols esters, mixtures thereof, and the like.

Block copolymers of ethylene and propylene oxide known as poloxamers are copolymers composed by a central hydrophobic chain of polyoxypropylen flanked by two hydrophilic chains of polyoxyethynene. Poloxamers are defined by a three-digit number, wherein the first two digits multiplied by 100 gives the approximate molecular mass of the polyoxypropylene core, and the last digit multiplied by 10 gives the percentage polyoxyethylene content. Examples of suitable poloxamers are poloxamer 124, 188, 237, 331, 338, 407 and the like. Examples of commercial block copolymers of ethylene and propylene oxide are Kolliphor® P188, Kolliphor® P188 micro, Kolliphor® P237, Kolliphor® P338, Kolliphor® P407, Kolliphor® P407 micro, Synperonic® PE/F 127, Synperonic® PE/F 68, Synperonic® PEIF 108, Synperonic® PE/F 44, and Synperonic® PEIF 101.

Fatty acid esters are esters of one fatty acid (fatty acids are linear or branched, saturated or insaturated carboxylic acids from 10 to 22 carbons) esterified to an alcohol moiety, including polyols such as glycol. Examples of suitable fatty acid ester are glyceryl monooleate (such as Capmul GMO-50 or CremerCOOR® GMO 90), glyceryl monostearate (such as Capmul GMS-50K or Cutina® GMS V), glyceryl caprylate (such as Capmul MCM C8 or Capmul MCM C8 EP), glyceryl caprate (such as Capmul MCM C10), and the like.

A medium-chain triglyceride is a glycerol moiety esterified with three medium-chain fatty acids. Medium chain fatty acids are considered fatty acids with of 6 to 12 carbons atoms in length, such as caproic acid, enanthic acid, caprylic acid, pelargonic acid, capric acid, undecylic acid or lauric acid. Examples of medium-chain triglycerides are caprylic triglyceride, caprylic/capric triglyceride and like. Commercial examples of medium-chain triglyceride are Miglyol® 812, Bergabest® MCT Oil 60/40, Bergabest® MCT Oil 70/30, Bergabest® MCT Oil 8/100, Captex® 355, Captex® 355 EP/NF, Captex® 355 EP/NF/JPE, Captex® 355 low C6, Crodamol® GTCC-PN, Labrafac® CC, Labrafac® Lipophile WL1349; Myritol® 318, Myritol® 812, Myritol® 312, Neobee® 895, Neobee® 1053, and Waglinol® 3/9280.

Cyclodextrins is a family of molecules made up of sugar molecules bound together to form a ring. Examples of cyclodextrines are α-cyclodextrin, β-cyclodextrin, γ-cyclodextrin, 2-hydroxypropyl-β-cyclodextrin, methyl-β-cyclodextrin, dimethyl-β-cyclodextrin, 2-hydroxyethyl-β-cyclodextrin, sulfobutylether-β-cyclodextrin, trimethyl-β-cyclodextrin and the like. Examples of commercial cyclodextrins are Cavitron W7 HP5 Pharma cyclodextrin, Cavitron W7 HP7 Pharma cyclodextrin, Cavamax W6 Pharma, Cavamax W7 Pharma, Cavamax W8 Pharma, and Klepsose® DC.

Polyethylene glycols, also known as macrogols, PEG or polyethylene oxides, are polymers of ethylene oxide of different length. The different grades of polyethylene glycols are referred as PEG followed by a number stating the average molecular weight. Examples of suitable polyethylene glycols are PEG 200, PEG 300, PEG 400, PEG 540, PEG 600, PEG 900, PEG 1000, PEG 1450, PEG 1540, PEG 2000, PEG 3000, PEG 3350, PEG 4000, PEG 4600, PEG 8000, and the like. Examples of commercial polyethylene glycols are Kollisolv® PEG 300, Kollisolv® PEG 400, Renex ™ PEG 400, Renex ™ PEG 1500, Renex ™ PEG 4000, and Renex ™ PEG 6000.

Sorbitan alkyl esters are molecules derived from sorbitan esterified with one or more fatty acid. Suitable sorbitan alkyl esters are sorbitan monoisostearate, sorbitan monolaurate, sorbitan monooleate, sorbitan monopalmitate, sorbitan monostearate, sorbitan sesquiisostearate, sorbitan sesquioleate, sorbitan trilaurate, sorbitan trioleate, sorbitan tristearate and the like. Commercial examples are Sorbester® P17, Sorbirol® L, Hodag®, Lamesorb®, Liposorb®, Montane®, Nikkol®, Polycon®, Sorgon® or Span@ 20, Span@ 40, Span@ 60, Span@ 65, Span® 80, Span@ 85, and SP Span@ 60 MBAL.

Polyoxyethylene sorbitan fatty acid esters (a.k.a. polysorbates) are molecules derived from polyethylenglycolated sorbitan moieties esterified with one or more fatty acid. Suitable polyoxyethylene sorbitan fatty acid esters are polysorbate 20 (polyoxyethylene (20) sorbitan monolaurate), polysorbate 21 (polyoxyethylene (4) sorbitan monolaurate), polysorbate 40 (polyoxyethylene (20) sorbitan monopalmitate), polysorbate 60 (polyoxyethylene (20) sorbitan monostearate), polysorbate 61 (polyoxyethylene (4) sorbitan monostearate), polysorbate 65 (polyoxyethylene (20) sorbitan tristearate), polysorbate 80 (polyoxyethylene (20) sorbitan monooleate), polysorbate 81 (polyoxyethylene (5) sorbitan monooleate), polysorbate 85 (polyoxyethylene (20) sorbitan trioleate), and polysorbate 120 (polyoxyethylene (20) sorbitan monoisostearate). There two ways of referring to this family of compounds, "polysorbates" plus a number or "polyoxyethylene" plus a number and the fatty acid present. The number following 'polysorbate' is related to the type of fatty acid associated with the polyoxyethylene sorbitan part of the molecule. Monolaurate is indicated by 20, monopalmitate is indicated by 40, monostearate by 60 and monooleate by 80. When the second digit is a zero, polysorbates includes a total of 20 units of oxyethylene $-(CH_2CH_2O)-$. When the second digit is not a zero polysorbates includes a total of oxyethylene $-(CH_2CH_2O)-$ units different from 20. In the second way of referring to these compounds the number following 'polyoxyethylene' refers to the total number of oxyethylene $-(CH_2CH_2O)-$ units found in the molecule. Commercial examples are Tween® 20, Tween® 21, Tween® 40, Tween® 60, Tween® 61, Tween® 65, Tween® 80, Tween® 81, Tween® 85, Kolliphor® PS 20, Kolliphor® PS 60, and Kolliphor® PS 80.

Polyoxylglycerides are mixtures of monoesters, diesters, and triesters of glycerol, and monoesters and diesters of polyethylene glycols (PEG). The polyoxyethylene moiety is commonly referred to as macrogol or polyoxyl and a digit giving the number of ethylene oxide units. Suitable examples of polyoxylglycerides are caprylocaproyl macrogolglycerides (such as Labrasol®), lauroyl macrogolglycerides (such as Gelucire® 44/14, and the like), linoleoyl macrogolglycerides (such as Labrafil® M2125CS, oleoyl macrogolglycerides (such as Labrafil® M1944CS), polyethylene glycol monocetyl ester, stearoyl macrogolglycerides (such as Gelucire® 50/13), and the like.

Polyoxyethylene fatty alcohol ethers are molecules made up of a fatty alcohol etherified to a polyethylene glycol of a given length, such as polyethylene glycol monooleyl ether, polyethylene glycol monolauryl ether, polyethylene glycol monostearyl ether and the like. Commercial examples are members of the Brij® family such as Brij® 02, Brij® O10, Brij® CS17, Brij® S2, Brij® S10, Brij® S2, Brij® C20, Brij® L23, Brij® IC20, Brij® LT3, and Brij® CS25.

Polyoxyethylene castor oil derivatives are molecules of castor oil etherified with polyethylene glycol. Examples of polyoxyethylene castor oil derivatives are macrogolglycerol ricinoleate, polyoxyl 35 castor oil, macrogolglycerol hydroxystearate, PEG-40 hydrogenated castor oil, PEG-35 castor oil, polyoxyl castor oil (such as Kolliphor® RH 40 or Kolliphor® EUELP), and the like.

Polyoxyethylene alkyl esters are esters of polyoxyethylene alcohol and an alkyl carboxylic acid. Suitable polyoxyethylene fatty acid esters are polyoxyethylene stearates esters such as polyoxyl 2, 4, 6, 8, 12, 20, 30, 40, 50, 100 or 150 stearates; polyoxyethylene 12, 32 and 150 distearates and the like. Commercial examples are Protamate® 200-OC (PEG-4 oleate), Protamate® 200-DPS (PEG-4 stearate), Protamate® 300-DPS (PEG-6 stearate), Protamate® 400-DPS (PEG-8 stearate), Protamate® 600-DPS (PEG-12 stearate), Protamate® 1540-DPS (PEG-40 stearate), Protamate® 2000-DPS (PEG-40 stearate), Protamate® 4400-DPS (PEG-100 stearate), Protamate® 200-ML (PEG-4 laurate), Protamate® 400-ML (PEG-8 laurate), Protamate® 600-ML (PEG-15 laurate), Protamate® 400-DO (PEG-8 dioleate), Protamate® 200-DS (PEG-4 distearate), Protamate® 400-DS (PEG-8 distearate), Protamate® 600-DS (PEG-12 distearate), Protamate® 6000-DS (PEG-150 distearate), Protamate® 200-DL (PEG-8 dilaurate), and Protamate® 400-DL (PEG-8 dilaurate).

Polyvinyl caprolactam-polyvinyl acetate polyethylene glycol graft copolymer is a copolymer consisting of a main polymer chain or backbone (polyethylene glycol) covalently bound to one or more side chains (copolymer of vinylcaprolactam and vinyl acetate) such as a graft-copolymer of PEG 6000 with vinylcaprolactam, and vinyl acetate commercially available as Soluplus®.

Polypropylene glycols esters are molecules made up of polypropylene glycol moiety esterified with fatty acids. Suitable examples are propylene glycol dicaprylate, propylene glycol dicaprate, propylene glycol dicaprylocaprate (such as Labrafac™ PG), and the like.

In a particular embodiment, in combination with any of the embodiments of the invention, the oral capsule of the invention is that in which the non-ionic surfactant of the atorvastatin tablet comprises a polyoxyethylene sorbitan fatty acid ester.

In another particular embodiment, in combination with any of the embodiments of the invention, the oral capsule of the invention is that in which the polyoxyethylene sorbitan fatty acid ester of the atorvastatin tablet is selected from the group consisting of: polysorbate 20, polysorbate 40, polysorbate 60, polysorbate 80, and mixtures thereof.

In another particular embodiment, in combination with any of the embodiments of the invention, the oral capsule of the invention is that in which the polyoxyethylene sorbitan fatty acid ester of the atorvastatin tablet is polysorbate 80.

In addition to atorvastatin or pharmaceutically acceptable salts thereof, non-ionic surfactant, and granulated calcium carbonate, atorvastatin tablets according to the present invention can generally contain other pharmaceutically acceptable excipients such as binders, diluents, lubricants, disintegrating agents, fillers, stabilizers, other surfactants, coloring agents, antiadhesive agents (gliding agents), coating agents and the like. The terms "pharmaceutically acceptable excipients", "pharmaceutically compatible excipients", and "excipients" are used interchangeably in this disclosure. They refer to non-API substances such as binders, diluents, lubricants, disintegrating agents, fillers, stabilizers, surfactants, coloring agents, antiadhesive agents (gliding agents), coating agents and the like used in formulating pharmaceutical products. They are generally safe for administering to humans according to established governmental standards.

Binders are used to impart cohesive qualities to a dosage form. Suitable binder materials include, but are not limited to, starch (including corn starch and pregelatinized starch), gelatin, sugars (including sucrose, glucose, dextrose, and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, ethyl cellulose, hydroxyethyl cellulose, and the like), and water-washed smectite clays such as Veegum®.

Diluents are typically used to increase bulk so that a practical size dosage unit, for example a tablet, is ultimately obtained. Suitable diluents include alkali metal carbonates, cellulose derivatives (microcrystalline cellulose, cellulose acetate, etc.), phosphates, maltodextrin, dextrin, fructose, dextrose, glyceryl palmitostearate, lactitol, lactose, including direct compression lactose, maltose, mannitol, sorbitol, starch, talc, xylitol, and/or hydrates thereof, and/or derivatives thereof.

Examples of suitable lubricants include, for example, metallic stearates (such as magnesium stearate, calcium stearate, aluminum stearate), fatty acid esters (such as sodium stearyl fumarate), fatty acids (such as stearic acid), fatty alcohols, glyceryl behenate, mineral oil, paraffins, hydrogenated vegetable oil, leucine, polyethylene glycols (PEG), metallic lauryl sulfate (such as sodium lauryl sulfate, magnesium lauryl sulfate), sodium chloride, sodium benzoate, sodium acetate, talc, siliconized talc, and/or hydrates thereof.

Disintegrating agents used in the present invention enable the dosage form to disperse in water easily and rapidly. Disintegrating agents can be selected from a group comprising polymers having high dispersing characteristics such as cross-linked hydroxypropyl cellulose, polyvinylpyrrolidone, high molecular weight polymers, microcrystalline cellulose, sodium starch glycolate, croscarmellose sodium, crospovidone; the products known under the trademarks Kollidon® CL, Polyplasdone®, alginic acid, sodium alginate, and corn starch.

Other basic agents that may be additionally used in the present invention are either watersoluble or water-insoluble and selected from a group comprising meglumine, tromethamol, sodium bicarbonate, sodium carbonate, sodium citrate, calcium gluconate, disodium hydrogen phosphate, dipotassium hydrogen phosphate, tripotassium phosphate, sodium tartrate, sodium acetate, calcium glycerophosphate, magnesium oxide, magnesium hydroxide, aluminum hydroxide, dihydroxy aluminum, sodium carbonate, calcium carbonate, aluminum carbonate, dihydroxy aluminum amino acetate, diethanolamine, triethanolamine, N-methyl-glucamine, glucosamine, ethylenediamine, triethyleneamine, isopropylamine, di-isopropyl amine, and combinations thereof.

Stabilizers are used to inhibit or retard drug decomposition reactions that include, by way of example, oxidative reactions.

Other surfactants that can optionally be used in the present invention are selected from the group consisting of anionic, cationic and amphoteric surfactants, such as sodium lauryl sulfate, dioctyl sulfosuccinate, gelatin, casein, lecithin, dextran. Sodium lauryl sulfate is a preferred optional surfactant according to the invention.

Antiadhesive agents (gliding agents) can be used in the present invention in order to prevent the mixture comprising active agents to adhere onto device and machine surfaces and create rough surfaces. The substances used for this purpose can comprise one or more components selected from a group comprising talc, colloidal silicone dioxide (Aerosil, Syloid, Cab-OSil), magnesium stearate, and corn starch.

Coating agents can be used to coat atorvastatin tablets or other active ingredients. For coating atorvastatin tablets coating agents not modifying the dissolution profile are preferred, such as conventional coatings based on HPMC or others well-known in the art.

Each atorvastatin tablet can preferably contain atorvastatin or pharmaceutically acceptable salts thereof in doses of 5 to 90 mg, more preferably in doses of 10 to 40 mg.

Aspirin (acetylsalicylic acid) may optionally be included in the capsules of the invention. Aspirin can be included for instance in the form of tablets, granules, beads, pellets, or powders.

The capsules of the invention can further comprise tablets, coated tablets, beads, pellets, granules or powders comprising an inhibitor of the rennin-angiotensin system as an active agent.

The renin aldosterone angiotensin system (RAAS) can be blocked at various levels. Renin inhibitors, ACE inhibitors (ACEi) and angiotensin receptor blockers (ARBs) represent major drug classes that block the RAAS. The renin inhibitors to which the present invention applies are any of those having renin inhibitory activity in vivo. In one embodiment, the renin inhibitor is preferably selected from aliskiren, ditekiren, terlakiren, zankiren, RO 66- 1132, RO 66-1168, VTP27999, ACT-280778, and TAK-272, and pharmaceutically acceptable salts, prodrugs, derivatives and isomers thereof. An ARB according to the invention is any molecule that can specifically antagonize or block the action of angiotensin II type 1 receptors (ATI receptors). ARBs suitable for use herein include, but are not limited to, losartan, valsartan, irbesartan, candesartan, telmisartan, eprosartan, tasosartan, zolarsartan, azilsartan, olmesartan, saprisartan, forasartan, E-4177, and ZD-8731, and pharmaceutically acceptable salts, prodrugs, derivatives, and isomers thereof.

In a preferred embodiment, the inhibitor of the rennin-angiotensin system is an angiotensin-converting enzyme (ACE) inhibitor. An ACEi according to the invention is any molecule that can specifically inhibit the enzymatic activity of angiotensin converting enzyme. ACEi suitable for use herein include, but are not limited to, benazepril, benazeprilat, captopril, zofenopril, enalapril, enaprilat, fosinopril, ceronapril, lisinopril, moexipril, perindopril, quinapril, ramipril, trandolapril, alacepril, cilazapril, delapril, imidapril, rentiapril, spirapril, temocapril, and moveltipril, and pharmaceutically acceptable salts, prodrugs, derivatives, and isomers thereof. In one embodiment, the preferred ACEi is ramipril and pharmaceutically acceptable salts thereof.

In a particular embodiment, ramipril is included in the capsules of the invention. Ramipril can also be included, for instance, in the form of tablets, granules, beads, pellets, or in powder form.

Ramipril is a prodrug, which is rapidly hydrolysed after absorption to the active metabolite ramiprilat. Ramipril and ramiprilat inhibit angiotensin-converting enzyme (ACE).

Similarity factor (f2) is discussed in point V. DISSOLUTION PROFILE COMPARISONS in 'Guidance for Industry: Dissolution Testing of Immediate Release Solid Oral Dosage Forms, U.S. Department of Health and Human Services, Food and Drug Administration, Center for Drug Evaluation and Research (CDER), August 1997'.

Similarity factor f2 is used to compare the similarity of two different oral solid products in terms of dissolution profile of the active ingredient. A test product (T) is compared with a reference drug (R, also called Reference Listed Drug or RLD). Here, the dissolution profile of atorvastatin tablets placed in capsules (Test) is compared to Cardyl® 80 mg film-coated tablets (Reference).

The model-independent similarity factor (f2) approach is a comparably simple and mostly accepted approach for comparing dissolution profiles. Most of the regulatory agencies demand the f2 as an aid to compare the bioavailability of generic drugs compared to reference listed drugs or innovators. The same applies to changes in the manufacturing process of a product. Similarity factor (f2) is calculated at different pH values.

f2 is highly appropriate for dissolution profile assessment when the comparison of 3 or more dissolution time points' needs to be done.

The dissolution measurements of the test and reference batches should be made under exactly the same conditions. The dissolution time points for both the profiles should be the same (e.g., 15, 30, 45, 60 minutes). Only one measurement should be considered after 85% dissolution of both the products.

Similarity factor (f2) measures comparison of percent (%) dissolution among two graphical lines (curves). It is a "Log-reciprocal-square-root conversion of the sum-of-squared-error".

$$f2= 50*Log(100/(1+\Sigma(R(t)-T(t))^2/n)^{1/2})$$

wherein n denotes number of time points,

R(t) indicated percentage release results of the reference lot (Reference) at time-point t, and

T(t) indicated percentage release results of the lot under evaluation (Test) at time.

Generally, f2 values greater than 50 (50-100) ensure sameness or equivalence of the two curves and, thus, of the performance of the test and reference products.

Similarity factor (f2) is used as a research tool to try to assess the chances of two products of showing approximately the same bioavailability, i.e., of being considered bioequivalent.

As illustrated in the examples, similarity factors (f2) found for the capsules of the invention (Example 1 Capsule and Example 4 Capsule) and the comparative capsules (Comparative Example 3 Capsule) when compared to the reference product (Cardyl 80 mg film coated tablets) are well below 50. This means that the dissolution profiles of the examples are not comparable to that of the reference product. Thus, based on these results, the expert could not have concluded that the test (examples) could have a bioavailability comparable to the reference product.

For instance, Comparative Example 3 Capsule shows a dissolution profile delayed in comparison to the reference product. However, Cmax for Comparative Example 3 Capsule is considerably higher than for the reference product (56.7 vs 45.6 ng/mL). Thus, Comparative Example 3 Capsule does not show bioavailability similar to that of the reference product. AUC 0-t for Comparative Example 3 Capsule is 205,0 vs 193.8 ng/mL*h.

Additionally, both Example 1 Capsule, Example 4 Capsule also show a clearly delayed dissolution profile at all pH values tested and similarity factors (f2) show also non similarity regarding the reference product (all values are clearly below 50 again). However, the pilot bioavailability study shows that both samples according to the invention show similar pharmacokinetic parameters (both for Cmax and $AUC_{0-t}$).

Cmax of Example 1 Capsules and Example 4 Capsules were similar to that of Cardyl® 80 mg film-coated tablet (46.2 and 47.8 vs 45.6 ng/mL).

AUC0-t of Example 1 Capsules and Example 4 Capsules were similar to that of Cardyl® 80 mg film-coated tablet (185.6 and 180.1 vs 193.8 ng/mL*h).

In a particular embodiment, in combination with any of the embodiments of the invention, the oral capsule of the invention is that in which the amount of atorvastatin (as atorvastatin free carboxylic acid) or pharmaceutically acceptable salts thereof ranges from 10 to 90 mg.

In another particular embodiment, in combination with any of the embodiments of the invention, the oral capsule of the invention is that in which atorvastatin tablets are prepared by wet granulation.

In another particular embodiment, in combination with any of the embodiments of the invention, the oral capsule of the invention is that in which the atorvastatin tablets comprise: a) an amount of 26-34 % weight/weight of atorvastatin or pharmaceutically acceptable salts thereof, b) an amount of 2.0-2.5 % weight/weight of a non-ionic surfactant, and c) an amount of 25-30 % weight/weight of granulated calcium carbonate.

In another particular embodiment, in combination with any of the embodiments of the invention, the oral capsule of the invention is that in which the atorvastatin tablets comprise sodium lauryl sulphate.

In another particular embodiment, in combination with any of the embodiments of the invention, the oral capsule of the invention is that in which the capsule comprises two atorvastatin tablets.

In another particular embodiment, in combination with any of the embodiments of the invention, the oral capsule of the invention is that in which atorvastatin tablets are coated tablets.

Obviously, the invention is not limited to the specific examples. The number of atorvastatin tablets of the examples can be modified. Atorvastatin tablets according to the invention could be placed in capsules together with other active ingredients in many other forms. For instance, ramipril could be replaced by other antihypertensive agent or agents including calcium channel blockers (such as amlodipine, nitrendipine, etc.), other ACE inhibitors (such as lisinopril or fosinopril), Angiotensin II receptor antagonists (such as losartan, valsartan, irbesartan, telmisartan), diuretics (such as hydrochlorothiazide, furosemide, etc.), or combinations thereof. Atorvastatin tablets could be placed in capsules together with other agents to treat dyslipidemias (such as ezetimibe, fenofibrate, niacin, etc.). All these other agents could be in any form, such as powder, tablets, granules, beads, or pellets.

In a particular embodiment, the capsule further comprises acetylsalicylic acid. In a further embodiment, acetylsalicylic acid is in the form of coated tablets.

In a particular embodiment, the capsule further comprises an inhibitor of the rennin-angiotensin system. In a further embodiment, the inhibitor of the rennin-angiotensin system is an ACE inhibitor. In a further embodiment, the ACE inhibitor is ramipril or a pharmaceutically acceptable salt thereof. In a further embodiment, acetylsalicylic acid and/or the inhibitor of the rennin-angiotensin system are present in the form of tablets, granules, beads or pellets. In a further embodiment, acetylsalicylic acid and/or the inhibitor of the rennin-angiotensin system are present in the form of tablets. In a further embodiment, the capsule of the invention comprises 2 tablets comprising acetylsalicylic acid, one tablet comprising ramipril, and one or two tablets comprising atorvastatin.

In a particular embodiment, capsules are hard-shelled capsule.

In a particular embodiment, capsules comprise gelatin or hydroxypropyl methylcellulose as ingredients of the capsule material.

In a particular embodiment, the locked length of the capsule ranges from 11.1 to 23.5 mm and the external diameter of the body of the capsule ranges from 4.68 to 8.2 mm. In a further embodiment, the locked length of the capsule ranges from 11.1 to 23.5 mm, and the external diameter of the body of the capsule ranges from 4.68 to 7.3 mm.

In a particular embodiment, the oral capsule according to the invention is that in which after being administered to healthy subjects under fast conditions at a dose of 80 mg of atorvastatin, the Cmax for atorvastatin or pharmaceutically acceptable salts thereof ranges from 40 ng/mL to 51 ng/mL and the $AUC_{0-t}$ ranges from 180 to 205 ng/mL*h or values proportional to the dose tested. The later means for instance that if a dose of 40 mg is tested, the values of Cmax and AUC0-t should be inside the ranges divided by two (as 40 mg is half of the 80 mg), i.e., Cmax for atorvastatin or pharmaceutically acceptable salts thereof the Cmax should range from 20 to 25.5 ng/mL and the AUC0-t should range from 90 to 102.5 ng/mL*h.

An atorvastatin tablet comprising: a) an amount of 25-35 % weight/weight of atorvastatin or pharmaceutically acceptable salts thereof, b) an amount of 2-3 % weight/weight of a non-ionic surfactant, and c) an amount of 20-35 % weight/weight of granulated calcium carbonate, wherein all amounts in % weight/weight are calculated in relation to the total weight of the atorvastatin tablet is also considered part of the invention.

In a particular embodiment, the atorvastatin tablet of the invention comprises: a) an amount of 26-34 % weight/weight of atorvastatin or pharmaceutically acceptable salts thereof, b) an amount of 2.0-2.5 % weight/weight of a non-ionic surfactant, and c) an amount of 25-30 % weight/weight of granulated calcium carbonate.

In another particular embodiment, in combination with any of the embodiments of the invention, the atorvastatin tablet of the invention is that in which the non-ionic surfactant comprises a polyoxyethylene sorbitan fatty acid ester.

In another particular embodiment, in combination with any of the embodiments of the invention, the atorvastatin tablet of the invention is that in which the polyoxyethylene sorbitan fatty acid ester is selected from the group consisting of: polysorbate 20, polysorbate 40, polysorbate 60, polysorbate 80, and mixtures thereof.

In another particular embodiment, in combination with any of the embodiments of the invention, the atorvastatin tablet of the invention is that in which the atorvastatin tablet has any of the additional features related to it according to any embodiment of the first aspect of the invention which is the oral capsule comprising the atorvastatin tablets.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular, further and preferred embodiments described herein.

**Examples**

*Atorvastatin tablets*

Atorvastatin tablets were prepared using crystalline atorvastatin calcium trihydrate in crystalline form I, showing a Dv90 < 50 microns and comprising 4.6 % of water determined by coulometry supplied by Ind-Swift Laboratories.

Atorvastatin tablets were manufactured by wet granulation using a high shear mixer and a fluid bed dryer. A granulation solution was prepared by dispersing polysorbate 80, sodium lauryl sulphate (if present) and hydroxypropyl cellulose in purified water. The rest of the intragranular components (see the following Table) were incorporated into the high shear mixer to be granulated. The granules were dried in the fluid bed dryer and sieved in an oscillating sieving machine. Finally, the extragranular components (see the following Table) were added and blended with the obtained granules. Each blend was compressed in a rotary press machine to produce round biconvex tablets. The diameter of the dies and punches was of 6 mm.

| Component | Example 1 Dose (mg) | Example 1 % w/w | Example 2 Dose (mg) | Example 2 % w/w | Example 3 Dose (mg) | Example 3 % w/w | Example 4 Dose (mg) | Example 4 % w/w | Comparative Example 1 Dose (mg) | Comparative Example 1 % w/w | Comparative Example 2 Dose (mg) | Comparative Example 2 % w/w | Comparative Example 3 Dose (mg) | Comparative Example 3 % w/w | Comparative Example 4 Dose (mg) | Comparative Example 4 % w/w |
|---|---|---|---|---|---|---|---|---|---|---|---|---|---|---|---|---|
| Atorvastatin calcium trihydrate | 43,299 | 30,0 | 43,299 | 30,0 | 43,299 | 30,0 | 43,299 | 30,0 | 43,299 | 30,0 | 43,299 | 30,0 | 28,867 | 23,4 | 43,299 | 30,0 |
| Lactose monohydrate[1] | 25,751 | 17,9 | 25,751 | 17,9 | 25,751 | 17,9 | 24,851 | 17,2 | 25,751 | 17,9 | 24,368 | 16,9 | 31,683 | 25,6 | 24,851 | 17,2 |
| Partially pregelatinized maize starch[2] | 17,000 | 11,8 | 17,000 | 11,8 | 17,000 | 11,8 | 17,000 | 11,8 | 17,000 | 11,8 | 16,983 | 11,8 | 18,000 | 14,6 | 17,000 | 11,8 |
| Calcium carbonate[3] | | | | | | | | | 40,000 | 27,7 | 40,000 | 27,7 | 28,000 | 22,7 | 40,000 | 27,7 |
| Calcium carbonate granulated with 5 % maize starch[4] | | | 40,000 | 27,7 | | | | | | | | | | | | |
| Calcium carbonate granulated with 10 % maize starch[5] | 40,000 | 27,7 | | | | | 40,000 | 27,7 | | | | | | | | |
| Calcium carbonate granulated with 10 % pregelatinized starch[6] | | | | | 40,000 | 27,7 | | | | | | | | | | |
| Hydroxypropylcellulose[7] | 3,000 | 2,1 | 3,000 | 2,1 | 3,000 | 2,1 | 3,000 | 2,1 | 3,000 | 2,1 | 3,000 | 2,1 | 3,000 | 2,4 | 3,000 | 2,1 |
| Polysorbate 80[8] | 3,200 | 2,2 | 3,200 | 2,2 | 3,200 | 2,2 | 3,200 | 2,2 | 3,200 | 2,2 | 3,200 | 2,2 | 2,100 | 1,7 | 3,200 | 2,2 |
| Sodium lauryl sulphate[9] | | | | | | | 0,900 | 0,6 | | | 1,400 | 1,0 | 0,600 | 0,5 | 0,900 | 0,6 |
| Purified water | 30,000 | | 30,000 | | 30,000 | | 30,000 | | 30,000 | | 30,000 | | 25,000 | | 30,000 | |
| TOTAL GRANULATE | 132,25 | 91,7 | 132,25 | 91,7 | 132,25 | 91,7 | 132,25 | 91,7 | 132,25 | 91,7 | 132,25 | 91,7 | 112,25 | 90,8 | 132,25 | 91,7 |
| Crospovidone[10] | 6,000 | 4,2 | 6,000 | 4,2 | 6,000 | 4,2 | 6,000 | 4,2 | 6,000 | 4,2 | 6,000 | 4,2 | 6,000 | 4,9 | 6,000 | 4,2 |
| Colloidal silicon dioxide[11] | 1,000 | 0,7 | 1,000 | 0,7 | 1,000 | 0,7 | 1,000 | 0,7 | 1,000 | 0,7 | 1,000 | 0,7 | 1,000 | 0,8 | 1,000 | 0,7 |
| Magnesium sterarate[12] | 0,750 | 0,5 | 0,750 | 0,5 | 0,750 | 0,5 | 0,750 | 0,5 | 0,750 | 0,5 | 0,750 | 0,5 | 0,750 | 0,6 | 0,750 | 0,5 |
| TOTAL CORE | 140,00 | 97,1 | 140,00 | 97,1 | 140,00 | 97,1 | 140,00 | 97,1 | 140,00 | 97,1 | 140,00 | 97,1 | 120,00 | 97,1 | 140,00 | 97,1 |
| HPMC-based coating* | 4,200 | 2,9 | 4,200 | 2,9 | 4,200 | 2,9 | 4,200 | 2,9 | 4,200 | 2,9 | 4,200 | 2,9 | 3,600 | 2,9 | 4,200 | 2,9 |

| Component | Example 1 | | Example 2 | | Example 3 | | Example 4 | | Comparative Example 1 | | Comparative Example 2 | | Comparative Example 3 | | Comparative Example 4 | |
|---|---|---|---|---|---|---|---|---|---|---|---|---|---|---|---|---|
| | Dose (mg) | % w/w | Dose (mg) | % w/w | Dose (mg) | % w/w | Dose (mg) | % w/w | Dose (mg) | % w/w | Dose (mg) | % w/w | Dose (mg) | % w/w | Dose (mg) | % w/w |
| Purified water | 28,000 | | 28,000 | | 28,000 | | 28,000 | | 28,000 | | 28,000 | | 24,000 | | 28,000 | |
| TOTAL FILM-COATED TABLET | 144,20 | 100 | 144,20 | 100 | 144,20 | 100 | 144,20 | 100 | 144,20 | 100 | 144,20 | 100 | 123,60 | 100 | 144,20 | 100 |

1. Pharmatose® 200M
2. Starch 1500®
3. Scoralite®
4. Scoralite® DC 95% ST
5. Scoralite® DC 90% ST
6. Scoralite® DC 90% pST
7. Klucel EF
8. Tween 80
9. Kolliphor ® SLS Fine
10. Kollidon CL
11. Aerosil® Pharma and 12 LIGAMEDO MF-2-V

EP 4 299 063 B1

After tableting, tablet cores obtained were film coated with a ready-to-use conventional immediate-release coating based on low viscosity hypromellose (hydroxypropyl methylcellulose, HPMC) having the following composition:

| *HPMC-based coating | % |
| --- | --- |
| hypromellose (HPMC) | 50,39 |
| talc | 33,27 |
| triethyl citrate | 8,14 |
| titanium dioxide | 4,72 |
| povidone | 2,52 |
| iron oxide red | 0,77 |
| iron oxide yellow | 0,19 |

Atorvastatin film-coated tablets in the above examples are round and biconvex and have a diameter of approximately 6 mm. Thickness of the tablets varies and is slightly dependent on the compression force applied: 40 mg atorvastatin film-coated tablets (comprising 43.3 mg of atorvastatin calcium trihydrate) have thickness of approximately 3.8 mm, while 26.7 mg atorvastatin film-coated tablets (comprising 28.9 mg of atorvastatin calcium trihydrate) of Comparative Example 3 have a thickness of approximately 3.3 mm.

### Capsules

Atorvastatin, aspirin and ramipril film-coated tablets were placed in hard gelatin capsules (Capsugel®). The size of capsules was #0 for all examples, except for Comparative Example 3 Capsule, in which capsules of size #0E were used.

Aspirin and ramipril film-coated tablets are also round and biconvex and have a diameter of approximately 6 mm. 50 mg aspirin film-coated tablets and 10 mg ramipril film-coated tablets have thickness of approximately 3 mm.

Aspirin tablets were those disclosed in Example 2 of WO 2014/195421 A1. Two 50 mg aspirin film-coated tablets with a coating thickness of 8.7 mg/cm2 (equivalent to a weight increase per tablet of 10 %) were placed in each capsule.

Ramipril tablets were 10 mg ramipril film-coated tablets prepared using the process disclosed in Example 9 of WO 2009/118359 A2. The composition of ramipril tablets was very similar to that disclosed in Table 29 in Example 9 of WO 2009/118359 A2 and is shown in the table below. A single 10 mg film-coated tablet was placed in each capsule.

| Ingredient | mg |
| --- | --- |
| Ramipril | 10.0 |
| Hypromellose 2910 (Methocel E5 Premium LV Hydroxypropyl Methylcellulose) | 2.0 |
| Partially pregelatinized maize starch (Starch 1500®) | 19.0 |
| Microcrystalline cellulose 200 (Vivapur® 200) | 69.5 |
| Sodium stearyl fumarate (Pruv®) | 0.5 |
| Partially hydrolised polyvinyl alcohol- based coating (Opadry® AMB) | 4.0 |

Number of tablets per capsule

| Capsule | # of tablets | | |
| --- | --- | --- | --- |
| | Atorvastatin | Aspirin | Ramipril |
| Example 1 Capsule | 2 | 2 | 1 |
| Example 4 Capsule | 2 | 2 | 1 |

(continued)

| Capsule | # of tablets | | |
|---|---|---|---|
| | Atorvastatin | Aspirin | Ramipril |
| Comparative Example 3 Capsule | 3 | 2 | 1 |
| Comparative Example 4 Capsule | 2 | 2 | 1 |

The examples of capsules include the atorvastatin tablets of the examples above. Thus, according to the table above, Example 1 Capsule contained 2 atorvastatin tablets according to the Example 1 above according to the invention and so on.

Thus, all the capsules of the examples contained a total dose of 80 mg of atorvastatin (equivalent to 86.60 mg of atorvastatin calcium trihydrate). Using the atorvastatin tablets disclosed in WO 2014/195421 A1, four atorvastatin tablets would be needed instead of 2. Thus, the invention enables the use of smaller capsules in comparison to those required in this prior art document for instance for including a total dose of 80 mg of atorvastatin.

Alternatively, capsules containing 40 mg of atorvastatin (equivalent to 43.30 mg of atorvastatin calcium trihydrate) could be manufactured using a single atorvastatin tablet according to the examples above according to the invention. For such capsules, the number of atorvastatin tablets used in each capsule is reduced from 2 in WO 2014/195421 A1 to just 1, enabling the use of smaller capsules or increasing the space available for further ingredients.

Additionally, alternatively capsules containing 20 mg of atorvastatin (equivalent to 21.69 mg of atorvastatin calcium trihydrate) having reduced volume could be manufactured using a single atorvastatin tablet following to the percentages of the invention. These capsules could be smaller as atorvastatin tablets will have a reduced size or the space available for further ingredients could be increased.

### *Dissolution profiles of tablets*

For efficiency of testing purposes, dissolution profiles shown for the tablets of the examples and comparative examples were carried out with tablet cores (uncoated tablets). Comparative tests were carried out with coated tablets and the dissolution profile was not modified by the coating of the tablets.

Dissolution profiles of tablets shown in the Figures were obtained from tests carried out in conventional dissolution testing equipment at a temperature of 37°C with agitation being provided by paddles (Apparatus 2) at 50 rpm according to the European Pharmacopoeia, 10.7, '2.9.3. Dissolution test for solid dosage forms'. Dissolution profiles were performed in three different dissolution media: pH 1.0 (0.1N HCl), pH 4.5 (acetate buffer) and pH 6.8 (phosphate buffer). The arithmetic mean of the values was obtained by testing 6 tablets of each type.

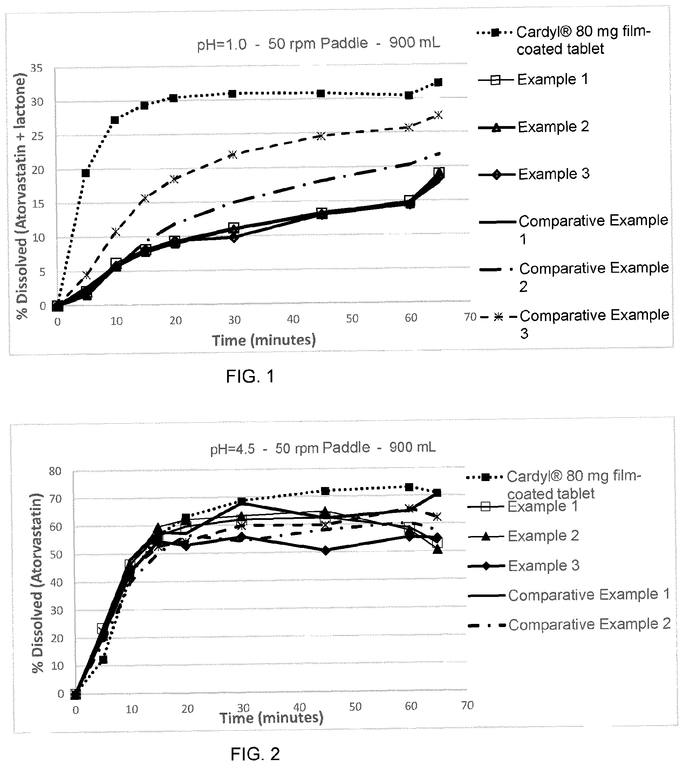

Figures 1, 2 and 3 show the dissolution profile of Examples 1-3 and Comparative Examples 1-3 tablet cores in comparison to the reference product (Cardyl 80 mg film-coated tablets) at pH values 1, 4.5 and 6.8 respectively at the conditions described above.

Examples 1-3 are identical in composition to Comparative Example 1, except for the fact that the latter comprises conventional calcium carbonate and Examples 1-3 comprise the same amount of granulated calcium carbonate. At pH values of 1 and 4.5 all these samples show similar dissolution profiles. However, at pH 6.8, unexpectedly, Examples 1-3 (comprising granulated calcium carbonate) show a faster dissolution profile compared to Comparative Example 1 (comprising standard calcium carbonate).

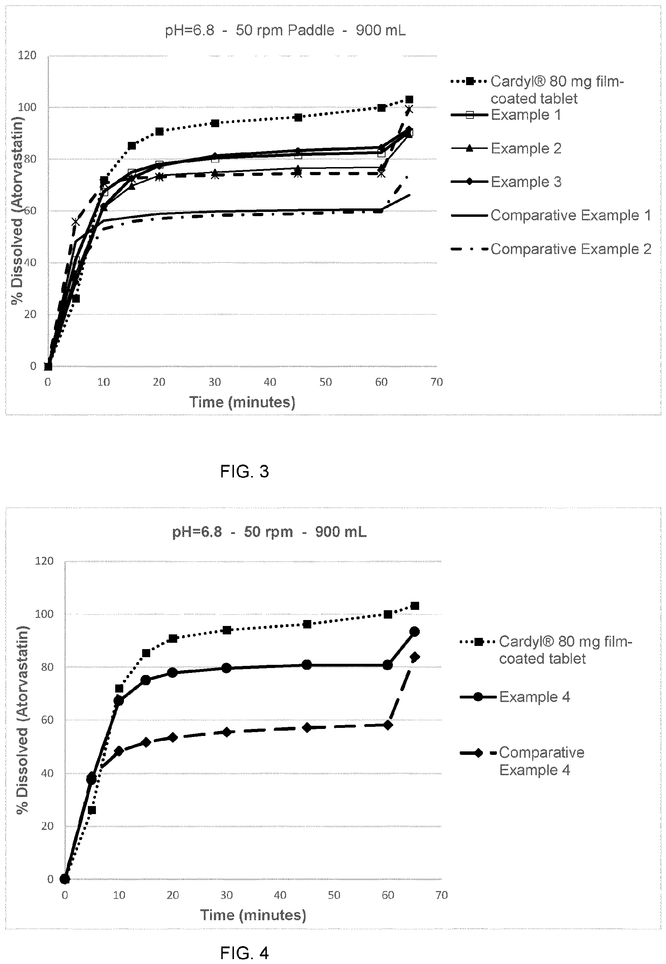

Comparative Example 2 (comprising standard calcium carbonate) has a similar composition to that of Examples 1-3 (comprising granulated calcium carbonate). Apart from the absence of granulated calcium carbonate, another difference is the presence of 1 % w/w of an anionic surfactant, sodium lauryl sulphate (Kolliphor ® SLS Fine). The presence of this additional surfactant does not accelerate the dissolution rate at pH=6.8 as shown in Figure 3.

Compared to Examples 1-3, Comparative Example 3 comprises a lower amount in % w/w of atorvastatin calcium (23.4 vs 30 %) and of nonionic surfactant per tablet (1.7 vs 2.2 %). At pH=1 Comparative Example 3 shows a faster dissolution profile compared to Examples 1-3. At pH=6.8 no major differences in dissolution profiler are shown compared to Examples 1-3.

Figure 4 shows the dissolution profile of Cardyl® 80 mg film-coated tablets, Example 4 and Comparative Example 4 tablets at pH 6.8 at 50 rpm and 900 mL. Example 4 and Comparative Example 4 have the same composition, except for the fact that Comparative Example 4 comprises conventional calcium carbonate, while Example 4 comprises calcium carbonate granulated with 10 % maize starch. Again, the presence of granulated calcium carbonate is associated with an acceleration in the dissolution profile as shown in Figure 4.

All Examples and Comparative Examples show a significantly delayed dissolution profile when compared to Cardyl® 80 mg film-coated tablet, a tablet wherein the amount of atorvastatin calcium is about 10.4 % w/w in relation to the total weight of the film-coated tablet.

***Dissolution profiles of capsules***

Dissolution profiles of capsules shown in the Figures were obtained from tests carried out in conventional dissolution testing equipment at a temperature of 37°C with agitation being provided by paddles (Apparatus 2) at different pH values and agitation conditions according to the European Pharmacopoeia, 10.7, '2.9.3. Dissolution test for solid dosage forms'. Dissolution profiles were performed in three different dissolution media: pH 1.0 (0.1N HCl), pH 4.5 (acetate buffer) and pH 6.8 (phosphate buffer). The arithmetic mean of the values of 6 capsules tested was considered.

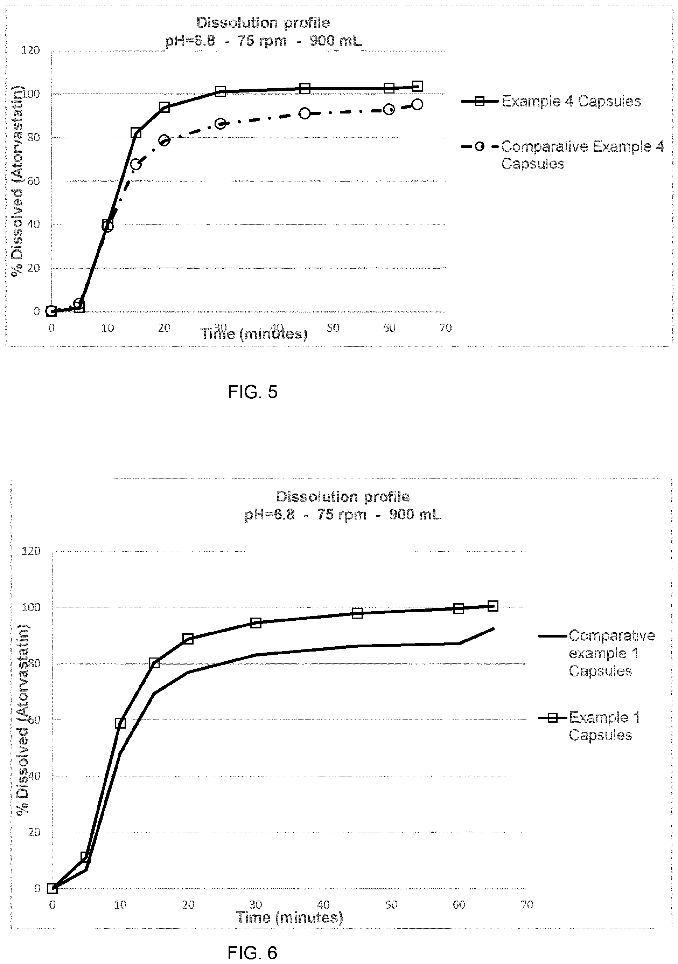

In Figure 5 the dissolution profiles of Example 4 Capsule and Comparative Example 4 Capsule at pH 6.8 at 75 rpm and 900 mL are shown. The same acceleration of the dissolution profile shown in tablets at pH=6.8 is shown for Example 4 Capsule comprising granulated calcium carbonate according to the invention.

The same effect is shown in Figure 6, where the dissolution profiles of Example 1 Capsule and Comparative Example 1 Capsule at pH= 6.8 at 75 rpm and 900 mL are shown.

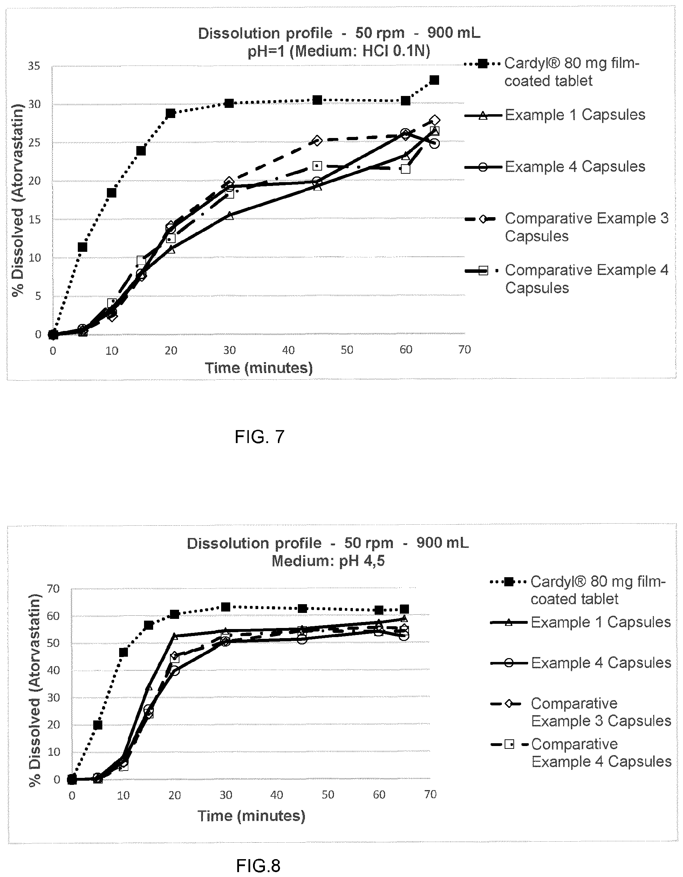

In Figures 7, 8 and 9 the acceleration of the dissolution profiles of the capsules of the invention at pH=6.8 is shown, as seen for tablets in Figures 1-3.

In Figure 7 the dissolution profiles of Example 1 Capsule, Example 4 Capsule, Comparative Example 3 Capsule and Comparative Example 4 Capsule at pH= 1.0, at 50 rpm and 900 mL are shown.

In Figure 8 the dissolution profiles of Example 1 Capsule, Example 4 Capsule, Comparative Example 3 Capsule and Comparative Example 4 Capsule at pH= 4.5 at 50 rpm and 900 mL are shown.

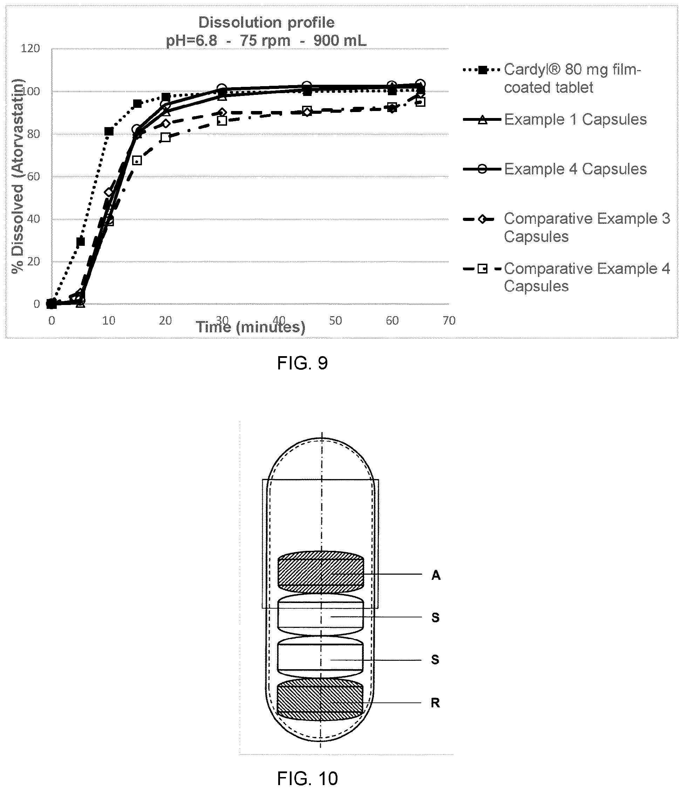

In Figure 9 the dissolution profiles of Example 1 Capsule, Example 4 Capsule, Comparative Example 3 Capsule and Comparative Example 4 Capsule at pH= 6.8, at 75 rpm and 900 mL are shown.

***Estimation of similarity factor (f2)***

Similarity factor (f2) was estimated using tests (capsules comprising atorvastatin tablets: Example 1 Capsule, Example 4 Capsule, Comparative Example 3 Capsule and Comparative Example 4 Capsule) and reference product (Cardyl® 80 mg film-coated tablets, commercially available). Using the mean dissolution values in percentage from both curves at each point in time, similarity factor (f2) was estimated.

The % of dissolution of atorvastatin was obtained at several pH values for several points in time with the equipment and under the conditions discussed above under 'dissolution profiles of capsules' and partially shown in the table below for each sample.

Following the recommendation found in 'Guidance for Industry: Dissolution Testing of Immediate Release Solid Oral Dosage Forms, U.S. Department of Health and Human Services, Food and Drug Administration, Center for Drug Evaluation and Research (CDER), August 1997', measurements of points in time were considered until the first point in time where 85% dissolution of both the test and reference products was achieved as detailed in the following table.

The % of atorvastatin dissolved of the capsules showed great variability in individual values at some points in time because the time a given capsule takes to be dissolved is highly variable. The mean % of dissolution of atorvastatin was used for the estimation of f2 also for points in time where the variability of individual values was high, because this is a reasonable alternative to use similarity factor (f2). This approach is justified by the nature of the sample.

| Similarity factor (f2) of capsules vs Cardyl® 80 mg film-coated tablets | | | | | | | | | |
|---|---|---|---|---|---|---|---|---|---|
| | | Example 1 Capsule | | Example 4 Capsule | | Comparative Example 3 Capsule | | Comparative Example 4 Capsule | |
| pH | rpm | f2 | No of points | f2 | No of points | f2 | No of points | f2 | No of points |
| 1.2 | 50 | 43 | 7 | 45 | 7 | 46 | 7 | 45 | 7 |
| 4.5 | 50 | 36 | 7 | 31 | 7 | 32 | 7 | 32 | 7 |
| 6.8 | 75 | 31 | 4 | 29 | 4 | 34 | 4 | 28 | 5 |

| No. of points | Points in time (min) |
|---|---|
| 4 | 5, 10, 15 and 20 |

(continued)

| No. of points | Points in time (min) |
|---|---|
| 5 | 5, 10, 15, 20 and 30 |
| 7 | 5, 10, 15, 20, 30, 45 and 60 |

None of the tested capsules showed values for the similarity factor (f2) at any pH in the range of 50-100. This means that the dissolution profiles are not similar in relation to the reference product and that it is not foreseeable from these results that the capsule products show similar bioavailability to that of Cardyl® 80 mg film-coated tablets. The results of this example are further discussed above in the Detailed description of the invention in relation to the comparative bioavailability study discussed below.

***Comparative bioavailability study***

Study title: A pilot, open label, cross-over, randomized, single dose comparative bioavailability study of distinct formulations of Polypill AAR 100/80/10 mg capsules Fixed dose combination: Comparative Example 3 Capsules, Example 1 Capsules, Example 4 Capsules versus equal doses of co-administered Cardyl® 80 mg film-coated tablets (REFERENCE formulation) in healthy male and female volunteers under fasting conditions.

Study objective: The present trial is a pilot comparative bioavailability study, performed to assess the potential for bioequivalence between distinct formulations: Comparative Example 3 Capsules, Example 1 Capsules, Example 4 Capsules versus equal doses of the co-administered Reference formulations (Cardyl® 80 mg film-coated tablet), each administered on one occasion (according to a cross-over study design) to male and female, healthy volunteers, under fasting conditions. Another significant objective was the assessment of safety following administration of study treatments and comparison of tolerability profiles. The potential for bioequivalence was assessed based on plasma drug levels of atorvastatin.

Study design: Pilot, open label, cross-over, block-randomized, single dose comparative bioavailability study on healthy male and female volunteers under fasting conditions.

Sample size: 35 subjects

Investigational products:

Tests products: Polypill AAR 100/80/10 mg capsules Comparative example 3 Capsules, Polypill AAR 100/80/10 mg capsules Example 1 Capsules, and Polypill AAR 100/80/10 mg capsules Example 4 Capsules manufactured by Ferrer International, S.A.

Reference product: Cardyl® 80mg film-coated tablets manufactured by Pfizer Manufacturing Deutschland GmbH.

Drug administration: Each volunteer received in a random way, a single oral dose of either TEST fixed-dose combination (Comparative Example 3 Capsules, Example 1 Capsules, Example 4 Capsules or the co-administered REFERENCE formulations in fasting conditions, each of them in one occasion, according to a cross-over study design. The study periods were separated by washouts of at least 14 days between one administration and the next one. The study products were administered orally with 240 ml of still bottled water at room temperature.

Blood sampling: During the treatment days, on all periods, about 5 mL venous blood samples were drawn in a labeled tube containing lithium heparin as anticoagulant at the following periods: before the administration and at 0.083 (5min.), 0.167 (10min.), 0.25 (15min.), 0.5 (30 min.), 0.667 (40min.), 0.833 (50min.), 1.0, 1.5 (1h 30min.), 2.0, 2.5 (2h 30min.), 3.0, 3.5 (3h 30min), 4.0, 6.0, 8.0, 10.0, 12.0, 24.0, 36.0 and 48.0 hours after treatment administration in each study period.

Bio-analytical methods: The identification and quantification of atorvastatin was performed by HPLC/MS/MS validated methods.

Pharmacokinetic results for atorvastatin:

| | Parameter | Mean | T/R (%) | LL (%) | UL (%) | CV (%) |
|---|---|---|---|---|---|---|
| **Comparative Example 3 Capsules** | Cmax | 56,676 | 122,806 | 101,75 | 148,22 | 43,383 |
| | AUC 0-t | 204,996 | 105,847 | 93,62 | 119,67 | 27,598 |
| **Example 1 Capsules** | Cmax | 46,227 | 103,826 | 91,46 | 117,87 | 28,559 |
| | AUC 0-t | 185,573 | 100,278 | 91,83 | 109,50 | 19,615 |
| **Example 4 Capsules** | Cmax | 47,810 | 107,359 | 89,70 | 128,50 | 42,972 |
| | AUC 0-t | 180,070 | 97,666 | 88,99 | 107,45 | 22,137 |
| **Cardyl® 80 mg film-coated tablet** | Cmax | 45,633 | - | - | - | - |
| | AUC 0-t | 193,840 | - | - | - | - |

Cmax is the maximum plasma concentration of atorvastatin expressed above in ng/mL as an arithmetic mean. AUC is the Area Under the plasma concentration-time Curve and reflects the extent of exposure to atorvastatin. AUC 0-t is the Area Under the plasma concentration-time Curve from time zero to the last measured timepoint using linear-log. trapezoidal rule expressed above in ng/mL*hour as an arithmetic mean. T/R is the ratio of the mean value for the Test (T) and the mean value for the Reference product (R) (expressed above as a percentage) calculated from log-transformed data. LL is the Lower Limit of the 90 % confidence interval of the ratio of the means of a log-transformed exposure measure (AUC and/or Cmax). UL is the Upper Limit of the 90 % confidence interval of the ratio of the means of a log-transformed exposure measure (AUC and/or Cmax). Coefficient of Variation (CV) is the ratio of the standard deviation to the mean.

Conclusions:

As a result of this study, it was concluded that Comparative Example 3 Capsule was not a suitable candidate comparing with Reference product whereas Example 1 Capsules and Example 4 Capsules were more favourable candidates as both prototypes Cmax and AUC showed potential to meet bioequivalence criteria in a pivotal study compared to the reference product.

In terms of safety, the three different atorvastatin treatments (Comparative Example 3 Capsules, Example 1 Capsules and Example 4 Capsules), administered in single dose, orally, to male and female adult healthy volunteers, in fasting conditions were well tolerated.

**Citation List**

Patent Literature

WO 2014/195421 A1

WO 2009/118359 A2

Non-Patent Literature

European Pharmacopoeia, 10.7, section '2.9.34. Bulk density and tapped density of powders'.

European Pharmacopoeia, 10.7, '2.9.3. Dissolution test for solid dosage forms'.

'Guidance for Industry: Dissolution Testing of Immediate Release Solid Oral Dosage Forms, U.S. Department of Health and Human Services, Food and Drug Administration, Center for Drug Evaluation and Research (CDER), August 1997'

**Claims**

1. An oral capsule comprising at least one atorvastatin tablet, wherein such atorvastatin tablets comprise:

   a. an amount of 25-35 % weight/weight of atorvastatin or a pharmaceutically acceptable salt thereof,
   b. an amount of 2-3 % weight/weight of a non-ionic surfactant, and
   c. an amount of 20-35 % weight/weight of granulated calcium carbonate,

   wherein all amounts in % weight/weight are calculated in relation to the total weight of the atorvastatin tablet.

2. The capsule according to the preceding claim, wherein the non-ionic surfactant comprises a polyoxyethylene sorbitan fatty acid ester.

3. The capsule according to the preceding claim, wherein the polyoxyethylene sorbitan fatty acid ester is selected from the group consisting of: polysorbate 20, polysorbate 40, polysorbate 60, polysorbate 80, and mixtures thereof.

4. The capsule according to the preceding claim, wherein the polyoxyethylene sorbitan fatty acid ester is polysorbate 80.

5. The capsule according to any of the preceding claims, wherein granulated calcium carbonate is calcium carbonate granulated with a binder in an amount equal to or less than 20 % weight/weight, wherein the % weight/weight is calculated in relation to the total weight of granulated calcium carbonate.

6. The capsule according to the preceding claim, wherein the granulated calcium carbonate comprises an amount of binder equal to or less than 10 % weight/weight, wherein the % weight/weight is calculated in relation to the total weight of granulated calcium carbonate.

7. The capsule according to any of the preceding claims, wherein the granulated calcium carbonate is calcium carbonate granulated with a binder selected from:
   maize starch, pregelatinized starch, potato starch, maltodextrin, sorbitol, polyvinyl pyrrolidone (PVP), acacia gum, methylcellulose, hydroxypropyl cellulose (HPC), hydroxypropyl methylcellulose (HPMC), sodium alginate (Na-Alg), gelatin, syrup, polyethylene glycol (PEG), a cationic copolymer based on dimethylaminoethyl methacrylate, butyl methacrylate and methyl methacrylate with a ratio of 2:1:1, and mixtures thereof.

8. The capsule according to any of the preceding claims, wherein the granulated calcium carbonate is calcium carbonate granulated with starch, pregelatinized starch, or mixtures thereof.

9. The capsule according to the preceding claim, wherein the granulated calcium carbonate comprises an amount of starch or pregelatinized starch of 5-10 % weight/weight.

10. The capsule according to any of the preceding claims, wherein the atorvastatin tablets comprise:

    a. an amount of 26-34 % weight/weight of atorvastatin or a pharmaceutically acceptable salt thereof,
    b. an amount of 2.0-2.5 % weight/weight of a non-ionic surfactant, and
    c. an amount of 25-30 % weight/weight of granulated calcium carbonate.

11. The capsule according to any of the preceding claims, which comprises two atorvastatin tablets.

12. The capsule according to any of the preceding claims, comprising acetylsalicylic acid.

13. The capsule according to any of the preceding claims, comprising an inhibitor of the rennin-angiotensin system.

14. The capsule according to the two preceding claims, wherein acetylsalicylic acid and/or the inhibitor of the rennin-angiotensin system are present in the form of tablets.

15. The capsule according to any of the preceding claims, wherein the locked length of the capsule ranges from 11.1 to 23.5 mm and the external diameter of the body of the capsule ranges from 4.68 to 8.2 mm.

**Patentansprüche**

1.  Kapsel zum Einnehmen, umfassend mindestens eine Atorvastatin-Tablette, wobei solche Atorvastatin-Tabletten Folgendes umfassen:

    A. eine Menge von 25-35 Gewichts-% Atorvastatin oder eines pharmazeutisch verträglichen Salzes davon,
    B. eine Menge von 2-3 Gewichts-% eines nichtionischen Tensids und
    C. eine Menge von 20-35 Gewichts-% granuliertem Calciumcarbonat,

    wobei alle Mengen in Gewichts-%, bezogen auf das Gesamtgewicht der Atorvastatin-Tablette, berechnet sind.

2.  Kapsel nach dem vorhergehenden Anspruch, wobei das nichtionische Tensid einen Polyoxyethylensorbitanfettsäureester umfasst.

3.  Kapsel nach dem vorhergehenden Anspruch, wobei der Polyoxyethylensorbitanfettsäureester ausgewählt ist aus der Gruppe, bestehend aus: Polysorbat 20, Polysorbat 40, Polysorbat 60, Polysorbat 80 und Mischungen davon.

4.  Kapsel nach dem vorhergehenden Anspruch, wobei der Polyoxyethylensorbitanfettsäureester Polysorbat 80 ist.

5.  Kapsel nach einem der vorhergehenden Ansprüche, wobei granuliertes Calciumcarbonat mit einem Bindemittel in einer Menge von gleich oder weniger als 20 Gewichts-% granuliertes Calciumcarbonat ist, wobei die Gewichts-% bezogen auf das Gesamtgewicht des granulierten Calciumcarbonats berechnet werden.

6.  Kapsel nach dem vorhergehenden Anspruch, wobei das granulierte Calciumcarbonat eine Menge an Bindemittel von gleich oder weniger als 10 Gewichts-% umfasst, wobei die Gewichts-% bezogen auf das Gesamtgewicht des granulierten Calciumcarbonats berechnet werden.

7.  Kapsel nach einem der vorhergehenden Ansprüche, wobei das granulierte Calciumcarbonat Calciumcarbonat ist, das mit einem Bindemittel granuliert ist, das ausgewählt ist aus: Maisstärke, vorgelatinierter Stärke, Kartoffelstärke, Maltodextrin, Sorbit, Polyvinylpyrrolidon (PVP), Akaziengummi, Methylcellulose, Hydroxypropylcellulose (HPC), Hydroxypropylmethylcellulose (HPMC), Natriumalginat (Na-Alg), Gelatine, Sirup, Polyethylenglykol (PEG), einem kationischen Copolymer auf der Basis von Dimethylaminoethylmethacrylat, Butylmethacrylat und Methylmethacrylat im Verhältnis 2:1:1 und Mischungen davon.

8.  Kapsel nach einem der vorhergehenden Ansprüche, wobei das granulierte Calciumcarbonat mit Stärke, vorgelatinierter Stärke oder Mischungen davon granuliertes Calciumcarbonat ist.

9.  Kapsel nach dem vorhergehenden Anspruch, wobei das granulierte Calciumcarbonat eine Menge an Stärke oder vorgelatinierter Stärke von 5-10 Gewichts-% umfasst.

10. Kapsel nach einem der vorhergehenden Ansprüche, wobei die Atorvastatin-Tabletten Folgendes umfassen:

    A. eine Menge von 26-34 Gewichts-% Atorvastatin oder eines pharmazeutisch verträglichen Salzes davon,
    B. eine Menge von 2,0-2,5 Gewichts-% eines nichtionischen Tensids und
    C. eine Menge von 25-30 Gewichts-% granuliertem Calciumcarbonat.

11. Kapsel nach einem der vorhergehenden Ansprüche, die zwei Atorvastatin-Tabletten umfasst.

12. Kapsel nach einem der vorhergehenden Ansprüche, umfassend Acetylsalicylsäure.

13. Kapsel nach einem der vorhergehenden Ansprüche, umfassend einen Inhibitor des Renin-Angiotensin-Systems.

14. Kapsel nach den beiden vorhergehenden Ansprüchen, wobei Acetylsalicylsäure und/oder der Inhibitor des Renin-Angiotensin-Systems in Form von Tabletten vorliegen.

15. Kapsel nach einem der vorhergehenden Ansprüche, wobei die verschlossene Länge der Kapsel von 11,1 bis 23,5 mm reicht und der Außendurchmesser des Kapselkörpers von 4,68 bis 8,2 mm reicht.

**Revendications**

1. Capsule orale comprenant au moins un comprimé d'atorvastatine, dans laquelle de tels comprimés d'atorvastatine comprennent :

   a. une quantité de 25 à 35 % en poids/poids d'atorvastatine ou d'un sel pharmaceutiquement acceptable de celle-ci,
   b. une quantité de 2 à 3 % en poids/poids d'un tensioactif non ionique et
   c. une quantité de 20 à 35 % en poids/poids de carbonate de calcium granulé, toutes les quantités en % en poids/poids étant calculées par rapport au poids total du comprimé d'atorvastatine.

2. Capsule selon la revendication précédente, dans laquelle le tensioactif non ionique comprend un ester d'acide gras de sorbitane polyoxyéthylénique.

3. Capsule selon la revendication précédente, dans laquelle l'ester d'acide gras de sorbitane polyoxyéthylénique est choisi dans le groupe constitué par : le polysorbate 20, le polysorbate 40, le polysorbate 60, le polysorbate 80 et les mélanges de ceux-ci.

4. Capsule selon la revendication précédente, dans laquelle l'ester d'acide gras de sorbitane polyoxyéthylénique est le polysorbate 80.

5. Capsule selon l'une quelconque des revendications précédentes, dans laquelle le carbonate de calcium granulé est du carbonate de calcium granulé avec un liant en une quantité inférieure ou égale à 20 % en poids/poids, le % en poids/poids étant calculé par rapport au poids total de carbonate de calcium granulé.

6. Capsule selon la revendication précédente, dans laquelle le carbonate de calcium granulé comprend une quantité de liant inférieure ou égale à 10 % en poids/poids, le % en poids/poids étant calculé par rapport au poids total de carbonate de calcium granulé.

7. Capsule selon l'une quelconque des revendications précédentes, dans laquelle le carbonate de calcium granulé est du carbonate de calcium granulé avec un liant choisi parmi : l'amidon de maïs, l'amidon prégélatinisé, l'amidon de pomme de terre, la maltodextrine, le sorbitol, la polyvinylpyrrolidone (PVP), la gomme d'acacia, la méthylcellulose, l'hydroxypropylcellulose (HPC), l'hydroxypropylméthylcellulose (HPMC), l'alginate de sodium (Na-Alg), la gélatine, du sirop, du polyéthylèneglycol (PEG), un copolymère cationique à base de méthacrylate de diméthylaminoéthyle, de méthacrylate de butyle et de méthacrylate de méthyle en un rapport de 2:1:1 et les mélanges de ceux-ci.

8. Capsule selon l'une quelconque des revendications précédentes, dans laquelle le carbonate de calcium granulé est du carbonate de calcium granulé avec de l'amidon, de l'amidon prégélatinisé ou des mélanges de ceux-ci.

9. Capsule selon la revendication précédente, dans laquelle le carbonate de calcium granulé comprend une quantité d'amidon ou d'amidon prégélatinisé de 5 à 10 % en poids/poids.

10. Capsule selon l'une quelconque des revendications précédentes, dans laquelle les comprimés d'atorvastatine comprennent :

    a. une quantité de 26 à 34 % en poids/poids d'atorvastatine ou d'un sel pharmaceutiquement acceptable de celle-ci,
    b. une quantité de 2,0 à 2,5 % en poids/poids d'un tensioactif non ionique et
    c. une quantité de 25 à 30 % en poids/poids de carbonate de calcium granulé.

11. Capsule selon l'une quelconque des revendications précédentes, qui comprend deux comprimés d'atorvastatine.

12. Capsule selon l'une quelconque des revendications précédentes, comprenant de l'acide acétylsalicylique.

13. Capsule selon l'une quelconque des revendications précédentes, comprenant un inhibiteur du système rénine-angiotensine.

14. Capsule selon les deux revendications précédentes, dans laquelle l'acide acétylsalicylique et/ou l'inhibiteur du système rénine-angiotensine sont présents sous la forme de comprimés.

15. Capsule selon l'une quelconque des revendications précédentes, dans laquelle la longueur fermée de la capsule va de 11,1 à 23,5 mm et le diamètre externe du corps de la capsule va de 4,68 à 8,2 mm.

FIG. 1

FIG. 2

FIG. 3

FIG. 4

FIG. 5

FIG. 6

FIG. 7

FIG.8

FIG. 9

FIG. 10

**REFERENCES CITED IN THE DESCRIPTION**

**Patent documents cited in the description**

- WO 2009118359 A2

- WO 2014195421 A1